# EUROPEAN PATENT APPLICATION

(11) **EP 4 454 646 A1**
(43) Date of publication of application: **30.10.2024**
(21) Application number: 22911039.0
(22) Date of filing: 14.12.2022
(51) Int. Cl.: A61K 31/437, A61K 31/4406, A61K 31/4439, A61K 31/4523, A61K 31/454, A61K 31/4545, A61K 31/506, A61K 31/513, A61K 31/519, A61K 31/704, A61K 31/7068, A61K 47/54, A61P 35/00, A61P 43/00, C07D 401/12, C07D 401/14, C07D 417/14, C07D 471/04, C07D 487/04

(54) **COMBINATION THERAPY INVOLVING RADIOTHERAPY AND HYPOXIA-RESPONSIVE PRODRUG OF ANTICANCER DRUG, AND NOVEL HYPOXIA-RESPONSIVE PRODRUG**

(30) Priority: 21.12.2021 JP 2021207215
(71) Applicant: University of Tsukuba, Ibaraki 305-8577 (JP)
(72) Inventor: IKEDA, Yutaka, Tsukuba-shi, Ibaraki 305-8577 (JP); NAGASAKI, Yukio, Tsukuba-shi, Ibaraki 305-8577 (JP)
(74) Representative: Kraus & Lederer PartGmbB
(86) International application number: PCT/JP2022/046076
(87) International publication number: WO 2023/120331

(57) **Abstract**

A prodrug of an anticancer drug is used in combination therapy with radiotherapy. In the chemical formula (1), a moiety in which R1 and R2 bond to an N atom is a part of an anticancer drug having an amino group or an imino group with the amino group or the imino group removed, and the anticancer drug is selected from the group consisting of gemcitabine, niraparib, crizotinib, tabrafenib, vemurafenib, entinostat, cobimetinib, and palbociclib, ribociclib. Thereby, the side effects of the treatment, including radiotherapy, for malignant tumors are reduced or the treatment effect is enhanced.

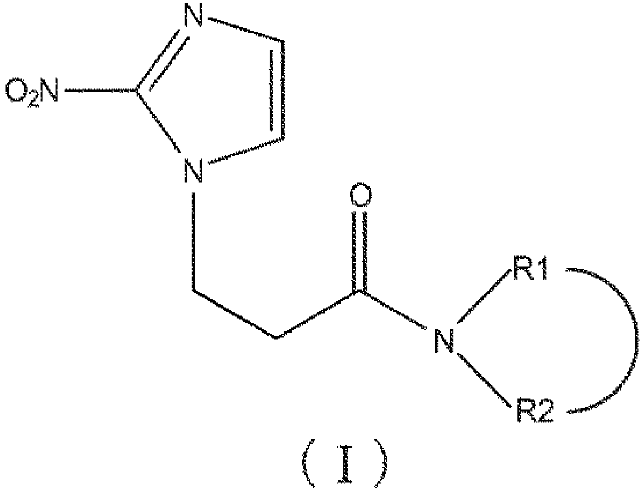

## Description

### Technical Field

The present invention relates to a formulation comprising a hypoxia-responsive prodrug as an active ingredient, for improving or enhancing the effect of radiotherapy in combination therapy of an anticancer drug and radiotherapy. More specifically, the present invention relates to the formulation in which the hypoxia-responsive prodrug is a compound having a structure covalently bonded to 2-nitro-1-imidazolepropionic acid through an amino group (-NH₂) or an imino group (-NH-) in a molecule of the anticancer drug by forming an amide or an imide, and to a novel hypoxia-responsive prodrug in the compound.

### Background Art

At present, radiotherapy with drug therapy (anticancer drug therapy) is recommended as a standard therapy for some types of cancer. In such combination therapy, both side effects associated with radiotherapy and side effects associated with anticancer drug(s) can occur. For this reason, for example, improvement in irradiation mode of irradiation and the like has been studied. Furthermore, in cells in a hypoxic state present in a malignant tumor, sensitization effect by oxygen may not be observed, or even if the effect is observed, it is very small. Thus, a cytotoxic effect of radiation on tumor cells is small. In general, a tumor including a large number of hypoxic cells becomes resistant to radiotherapy. Therefore, radiosensitizers for hypoxic cells and hypoxia-activated prodrugs have been proposed (I. N. Mistry et. al., Int J Radiation Oncol Bio Phys, Vol. 98, No. 5, pp. 1183-1196, 2017 (hereinafter referred to as Non-Patent Literature 1)). For example, nitroimidazole-based TH-302 (evofosafamide) has been proposed as a prodrug as described above, and combination therapy with radiotherapy has been investigated; however, as far as the present inventors know, it has not reached clinical use. In addition, the sensitizers as described above such as similar nitroimidazole-based pimonidazole, etanidazole, nimorazole and the like have been proposed; however, any of these compounds achieves no significant improvement statistically in survival rate compared to typical radiotherapy alone, and the combination of these compounds with radiotherapy either has been found no significant benefit or has not resulted in clinical use due to its higher toxicity. It should be noted that it is described that cancer can be treated by administering TH-302 alone or in combination with other anticancer agents and/or radiotherapy (however, no specific data regarding specific treatments combined with the therapy are disclosed and the results of the subsequent clinical trials and the like are against their expectation, as described in Non-Patent Literature 1.)

### Disclosure of the Invention

### Technical Problem

Nevertheless, there still exists a need to provide a compound which exerts more pronounced combined effects in combination therapy of radiotherapy and drug therapy.

Some of the present inventors have previously proposed a compound as a hypoxia-responsive prodrug of an anticancer drug. The compound belongs to a nitroimidazole-based compound, in which 2-nitro-1-imidazolepropionic acid is covalently bonded to the anticancer drug via an amino group (-NH₂) or an imino group (-NH-) in a molecule of the anticancer drug (Japanese Patent JP 5676020 B2). Such a compound (hereinafter sometimes abbreviated as PropHAPs) may contribute to the drug therapy for cancer in that it exhibits cytotoxicity comparable to that of a parent anticancer drug against malignant tumor cells at a low oxygen concentration but exhibits significantly lower cytotoxicity than that of the parent anticancer drug in cells at normal oxygen concentration.

In fact, as described in Non-Patent Literature 1, the nitroimidazole-based prodrugs described therein did not necessarily exhibit satisfactory effects in combination therapy with radiotherapy performed by us. Nevertheless, we investigated if there exists benefit or not in the therapy combined with radiotherapy using the PropHAP of gemcitabine. As a result, it was unexpectedly confirmed that the PropHAP of gemcitabine, in the therapy combined with radiotherapy (in vivo), exhibits a therapeutic effect on experimental animals that is almost equivalent to that of the parent compound while not resulting in a significant weight loss, at a radiation dose at which the parent compound causes side effects such as weight loss in the subject animals. Even if the dose of the drug was increased, the effect stayed the same. On the other hand, in a case where the PropHAP was used at a radiation dose and a drug dose at which the parent compound does not substantially express a side effect represented by weight loss or the like in the subject animals, the combination of the radiotherapy and the drug therapy prevails significantly superior anticancer effects, compared to those of the radiation monotherapy, the parent compound monotherapy and the combination of the parent compound and the radiotherapy. Such effects were also observed in case of the PropHAP of palbociclib, niraparib, ribociclib or doxorubicin. Each PropHAP differs from gemcitabine in its chemical structure of the anticancer drug and the mechanism of action on tumors.

The PropHAP is different from the typical nitroimidazole-based HAP described above because it is understood that PropHAP undergoes cleavage of a residual group or moiety X derived from an anticancer drug at a low hypoxic environment as shown in the following reaction scheme as described in the above-mentioned patent publication. It is considered that the chemical structure is different from that of the nitroimidazole-based prodrug described in Non-Patent Literature 1 and both the mode of cleavage of the nitroimidazole-based protective group and the product obtained by cleavage are completely different. In view of them, in the present invention or the subject matter disclosed herein, a use of Prop, referred as PropHAPs, as a protective group for the parent compound is of great importance.

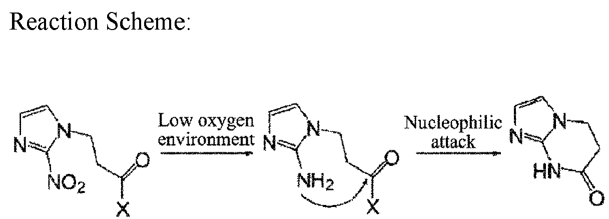

### Solution to Problem

Accordingly, main aspects or features of the invention provided the present application or the subject matter disclosed in the present description can include the following.

Aspect 1: A pharmaceutical formulation for improving or enhancing, or for use in improving or enhancing, a therapeutic effect of combination therapy including radiotherapy, in the combination therapy of radiotherapy and drug therapy for a malignant tumor, the pharmaceutical formulation comprising a compound represented by Formula I as an active ingredient: 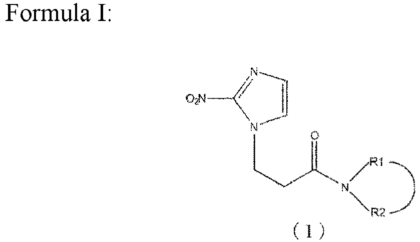 wherein,
a moiety in which R1 and R2 bond to an N atom is a part of an anticancer drug having an amino group or an imino group with the amino group or the imino group removed; in case of the anticancer drug having the amino group, either R1 or R2 represents a hydrogen atom, and the anticancer drug having the moiety in which R1 and R2 bond to an N atom is selected from the group consisting of: anthracyclines selected from doxorubicin, idarubicin, epirubicin, daunorubicin, pirarubicin, amrubicin, aclacinomycin, anthramycin and zorubicin; peptides selected from bleomycin and actinomycin; quinoline alkaloids selected from camptothecin, topotecan and irinotecan; taxanes selected from docetaxel and paclitaxel; vinca alkaloids selected from vinorelbine, vincristine, vinblastine and vindesine; deoxycytidines selected from gemcitabine and cytarabine; pyrimidines selected from 5-fluorouracil, capecitabine and doxifluridine; purine ring derivatives selected from fludarabine, 6-mercaptopurine and 6-thioguanine; macrolides selected from epothilone; and other anticancer drugs selected from niraparib, crizotinib, dabrafenib, vemurafenib, entinostat, panobinostat, cobimetinib, palbociclib and ribociclib.

Aspect 2: The pharmaceutical formulation according to Aspect 1, wherein the compound represented by Formula I is derived from the following anticancer drug and represented by the following structural formulae:
the compound being: derived from gemcitabine and represented by the structural formula: 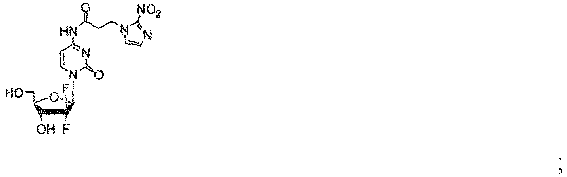
derived from doxorubicin and represented by the structural formula: 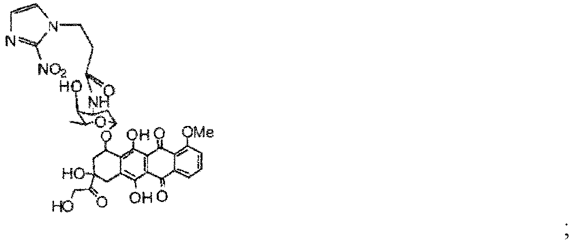
derived from 5-fluorouracil and represented by any one of the structural formulae: 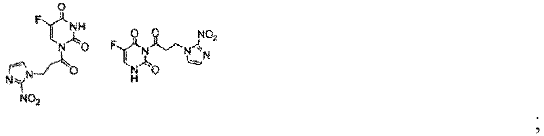
derived from niraparib and represented by the structural formula: 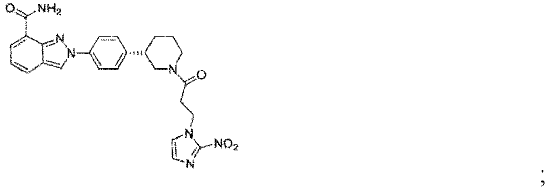
derived from crizotinib and represented by any one of the structural formulae: 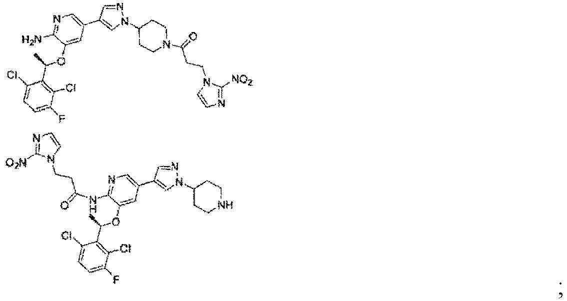
derived from dabrafenib and represented by the structural formula: 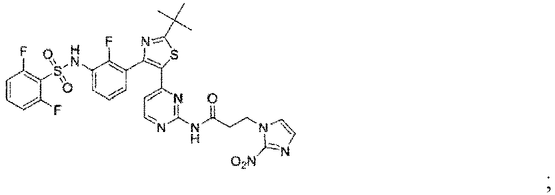
derived from vemurafenib and represented by the structural formula: 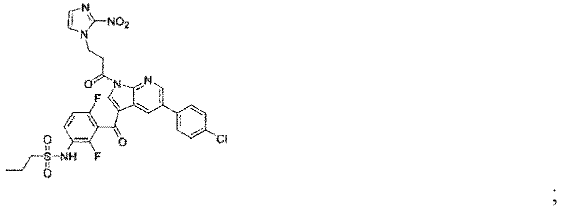
derived from entinostat and represented by the structural formula: 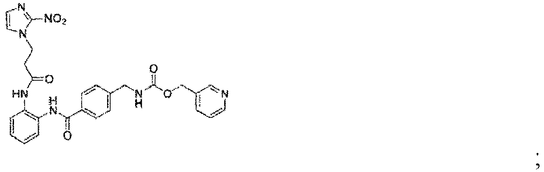
derived from cobimetinib and represented by the structural formula: 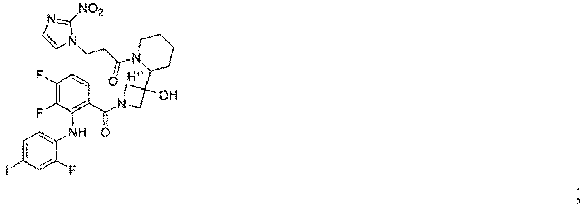
derived from palbociclib and represented by the structural formula: 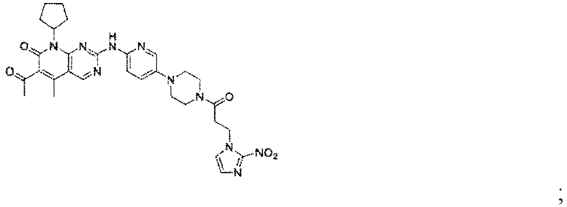 or
derived from ribociclib and represented by the structural formula:

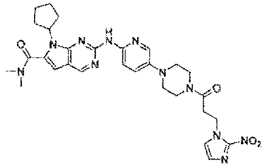

Aspect 3: Combination therapy of drug therapy and radiotherapy for a malignant tumor, the combination therapy comprising: administering an effective dose of a compound of Formula I to a patient or an individual in need of treatment for a malignant tumor; and irradiating the patient or the individual with an effective dose of radiation: 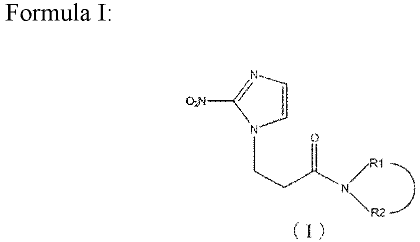 wherein,
a moiety in which R1 and R2 bond to an N atom is a part of an anticancer drug having an amino group or an imino group with the amino group or the imino group removed; in case of the anticancer drug having the amino group, either R1 or R2 represents a hydrogen atom, and the anticancer drug having the moiety in which R1 and R2 bond to an N atom is selected from the group consisting of: anthracyclines selected from doxorubicin, idarubicin, epirubicin, daunorubicin, pirarubicin, amrubicin, aclacinomycin, anthramycin and zorubicin; peptides selected from bleomycin and actinomycin; quinoline alkaloids selected from camptothecin, topotecan and irinotecan; taxanes selected from docetaxel and paclitaxel; vinca alkaloids selected from vinorelbine, vincristine, vinblastine and vindesine; deoxycytidines selected from gemcitabine and cytarabine; pyrimidines selected from 5-fluorouracil, capecitabine and doxifluridine; purine ring derivatives selected from fludarabine, 6-mercaptopurine and 6-thioguanine; macrolides selected from epothilone; and other anticancer drugs selected from niraparib, crizotinib, dabrafenib, vemurafenib, entinostat, panobinostat, cobimetinib, palbociclib and ribociclib.

Aspect 4: The combination therapy according to Aspect 3, wherein the compound represented by Formula I is derived from the following anticancer drug and represented by the following structural formulae:
the compound being: derived from gemcitabine and represented by the structural formula: 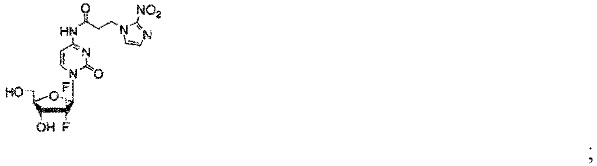
derived from doxorubicin and represented by the structural formula: 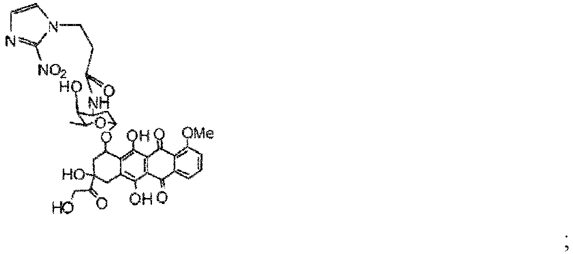
derived from 5-fluorouracil and represented by any one of the structural formulae: 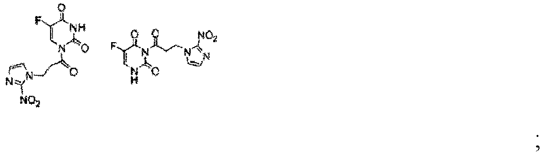
derived from niraparib and represented by the structural formula: 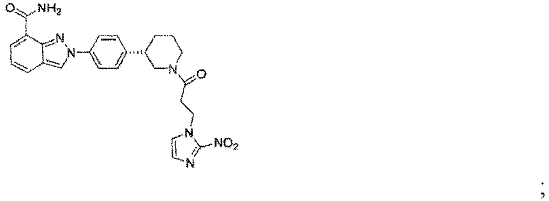
derived from crizotinib and represented by any one of the structural formulae: 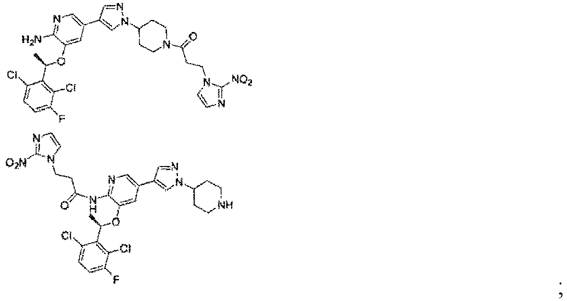
derived from dabrafenib and represented by the structural formula: 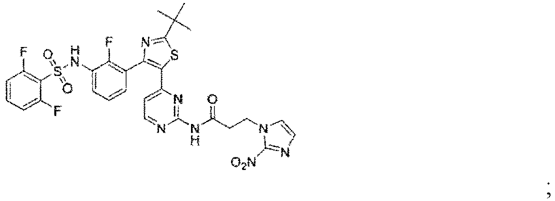
derived from vemurafenib and represented by the structural formula: 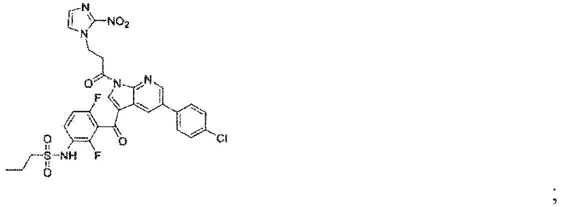
derived from entinostat and represented by the structural formula: 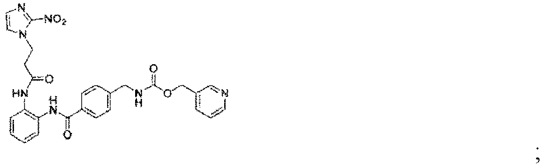
derived from cobimetinib and represented by the structural formula: 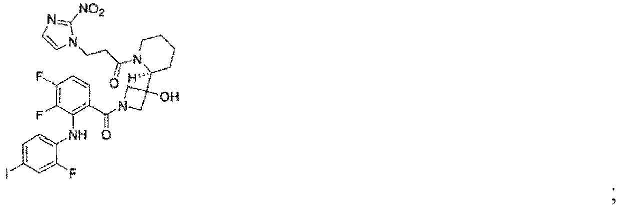
derived from palbociclib and represented by the structural formula: 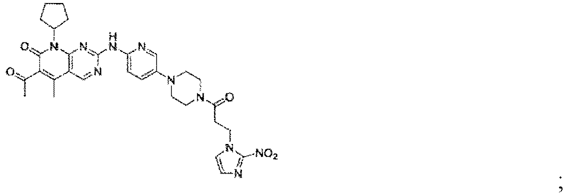 or
derived from ribociclib and represented by the structural formula :

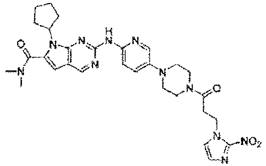

Aspect 5: A compound represented by Formula I-1 or a pharmaceutically acceptable salt thereof: 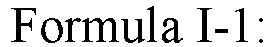 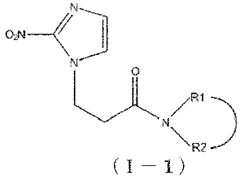 wherein,
a moiety in which R1 and R2 bond to an N atom is a part of an anticancer drug having an amino group or an imino group with the amino group or the imino group removed; and in case of the anticancer drug having the amino group, either R1 or R2 represents a hydrogen atom, and the compound is derived from the anticancer drug selected from the group consisting of niraparib, crizotinib, dabrafenib, vemurafenib, entinostat, cobimetinib, palbociclib and ribociclib; respectively, the compound being: derived from niraparib and represented by the structural formula: 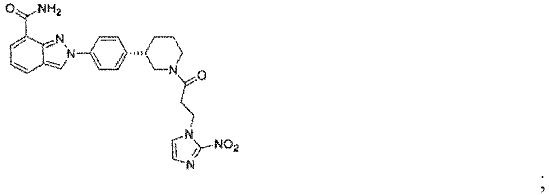
derived from crizotinib and represented by any one of the structural formulae: 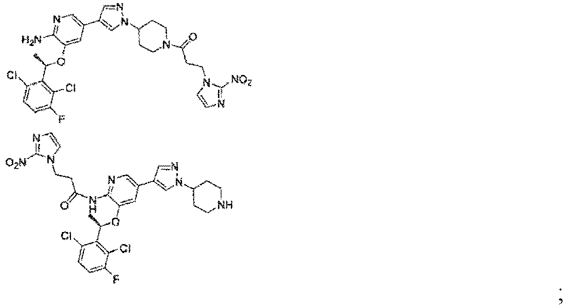
derived from dabrafenib and represented by the structural formula: 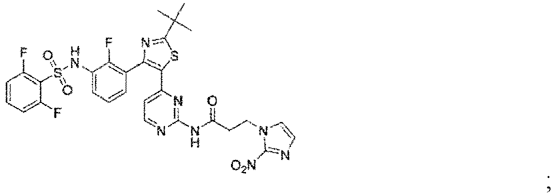
derived from vemurafenib and represented by the structural formula: 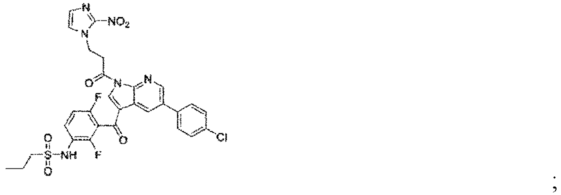
derived from entinostat and represented by the structural formula: 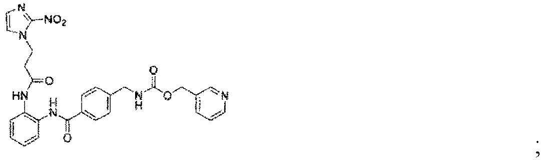
derived from cobimetinib and represented by the structural formula: 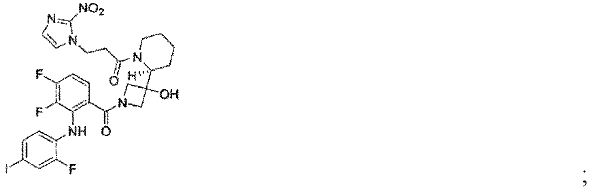
derived from palbociclib and represented by the structural formula: 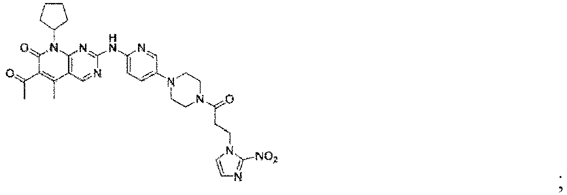 or
derived from ribociclib and represented by the structural formula:

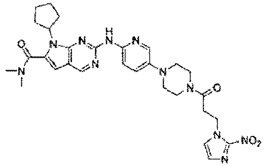

Aspect 6: A method of providing a hypoxia-responsive prodrug in combination therapy of radiotherapy and drug therapy for a malignant tumor, for improving or enhancing a therapeutic effect of combination therapy including the radiotherapy, the method comprising:
(1) covalently bonding an anticancer drug, via an amino group (NH₂) or an imino group (-NH-) with an imidazolepropionic acid represented by Formula II: 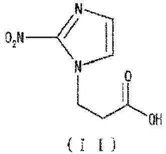 to form a prodrug of an anticancer drug, and
(2) treating a tumor experimental animal with the prodrug of the anticancer drug obtained in step (1) and its corresponding parent compound in combination with radiotherapy, to select the prodrug having statistically significantly lower side effects or significantly higher therapeutic effects higher than radiotherapy alone.

### Brief Description of Drawings

FIG. 1 is a graphical representation of the results of the (b) in vitro study 1 of Example 1.
FIG. 2 is a graphical representation of the results of the (c) in vitro study 2 of Example 1.
FIG. 3 is a graphical representation of the results of the (d) in vivo study of Example 1.
FIG. 4 is a graphical representation of the results of the (b) in vitro study of Example 2.
FIG. 5 is a graphical representation of the results of the (b) in vitro study of Example 3.
FIG. 6 is a graphical representation of the results of the (b) in vitro study of Example 4.
FIG. 7 is a graphical representation of the results of the (b) in vitro study of Example 5.
FIG. 8 is a graphical representation of the results of the (b) in vitro study of Example 6.
FIG. 9 is a graphical representation of the results of the (b) in vitro study of Example 7.
FIG. 10 is a graphical representation of the results of the (c) in vivo study of Example 7.
FIG. 11 is a graphical representation of the results of the (b) in vitro study and the (c) in vivo study of Example 8.
FIG. 12 is a graphical representation of the results of the in vivo study 1 of Example 9.
FIG. 13 is a graphical representation of the results of the in vivo study 2 of Example 9.
FIG. 14 is a graphical representation of the results of the in vivo study 2 (part 2) of Example 9.
FIG. 15 is a graphical representation of the results of the in vivo study 3 of Example 9.
FIG. 16 is a graphical representation of the results of the in vivo study of Example 10.

### Detailed Description of Invention

The technical terms used herein or used in relation to the description of the present invention have meanings and contents commonly used in the art, unless otherwise defined.

The radiation used in the "radiotherapy" of the present invention is one used in ordinary radiotherapy in a broad sense, and examples thereof can include, but are not limited to, X-ray, gamma (γ) ray, alpha (α) ray, beta (β) ray, proton beam and heavy particle (carbon ion) ray, and the irradiation mode of the radiation may be any mode already used in the art.

In the combination therapy of the invention, a patient or an individual (which may be a mammal, including a human) is generally subjected to irradiation from about 30 minutes to about 48 hours, typically from about 1 hour to about 24 hours after administration of the compound of Formula I. Irradiation can be external irradiation in which radiation is applied from outside the body of the patient or subject, internal irradiation in which radiation is applied to the cancer or its surroundings from an irradiation source embedded in the body, or a combination of both. In addition, malignant tumors that can be remedied or treated by such administration and irradiation include, for example, head and neck cancer, cervical cancer, esophageal cancer, lung cancer, pancreatic cancer, breast cancer, soft tissue sarcoma, and other tumors that may be treated by radiotherapy and drug administration or chemoradiotherapy at present or in the future, and for which treatment by said therapy is expected to provide the desired effect. On the other hand, the administration mode of the compound represented by Formula I may be oral administration or parenteral administration, and specifically, it can be appropriately determined with reference to the administration mode of being usually used in clinical cases for the parent compound.

The meaning of the phrase "improving or enhancing an effect of combination therapy including radiotherapy" is defined as follows: in combination therapy with radio therapy, when an experimental animal bearing a malignant tumor is subjected to administration of a parent compound of a compound represented by Formula I (an anticancer drug having a moiety in which R1 and R2 bonded to the N atom in the compound represented by Formula I, or a compound serving as a starting material of the compound) and then subjected to radiotherapy, the animal may exhibit nausea, vomiting, neuropathy and the like or weight loss and the like sometimes resulting therefrom. These adverse conditions are statistically and significantly reduced when the parent compound is replaced with the compound of Formula I, while the therapeutic effect is at least comparable or at least statistically and significantly enhanced as compared with that by radiotherapy alone. The phrase "statistically significant reduction" or "significant enhancement" means a reduction or enhancement of the effect with a statistically significant difference when data obtained from the study results of a group of five or more of subjects (including subject individuals) are statistically processed by t-test.

"Pharmaceutical formulation" is a formulation which can be used in the art of medicine and is a concept which is interchangeable with pharmaceutical composition and can comprise, in addition to the compound of Formula I as an active ingredient, diluents, carriers, excipients and the like. These are generally safe, non-toxic and do not adversely affect the properties of the compound and include but are not limited to sterile water, non-ionic surfactants, ethanol, glycerol and mixtures thereof and other compounds or mixtures which have been used as diluents, carriers or excipients for individual drug substances.

The pharmaceutically acceptable salt of the compound disclosed in Aspect 5 can be an acid addition salt of a mineral acid such as hydrochloric acid or sulfuric acid or an organic acid such as formic acid, acetic acid, citric acid or methanesulfonic acid. When the compound has an acidic group such as a carboxyl group or a hydroxyl group, it can be an addition salt of an alkali metal such as sodium or potassium or an organic amine such as ammonium or methylamine.

A compound of Formula I according to Aspect 1 or a hypoxia-responsive prodrug according to Aspect 6 may be prepared by reaction of a corresponding or general anticancer drug with an imidazolepropionic acid represented by Formula II to form an amide or imide bond. Such a reaction may be carried out by reacting the anti-cancer drug with the compound of Formula II in a suitable inert solvent in the presence of a condensing agent well known per se in the art (e.g. carbodiimides) or by reacting an active ester of the compound of Formula II (halide, ester of the compound with N-hydroxysuccinimide, etc.) with the compound of Formula I in a suitable solvent. As a specific example of the reaction, when an amino group or a cyclic amino group and a hydroxyl group coexist in the molecule of the organic compound, any one of the groups may be protected by a method known in the art if necessary, and then any one of the reactions described above may be carried out. For specific examples of carrying out the reaction, reference can be made to those described in Japanese Patent JP 5676020 B2, and for the PropHAPs of gemcitabine, doxorubicin and 5-fluorouracil, those described therein can be used as they are, so that the contents thereof are incorporated herein by reference in their entirety.

The effective dose of irradiation and the effective dose of the compound represented by Formula I cannot be specified because the optimal dose varies depending on the type and severity of the malignant tumor to be treated and the type of the compound to be used. However, the irradiation dose, usage and dosage of the corresponding parent compound which has been specifically examined for such combination therapy can be referred to; for example, they can be determined by a medical professional with reference to materials available from the United States Food and Drug Administration, data discussed in pharmaceutical literatures, and the like.

### Examples

Hereinafter, the present invention will be disclosed in more detail with reference to specific examples, but is not intended to limit itself to these examples.

### Example 1: Niraparib HAP

### (a) Example of production

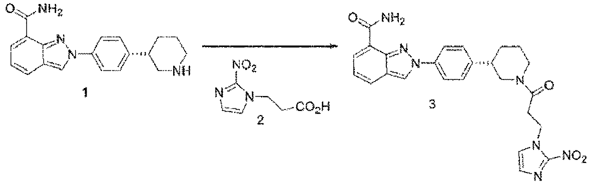

Niraparib (Compound 1, 100 mg) was dissolved in 10 mL of dichloromethane, Compound 2 (105 mg), 1-(3-dimethylaminopropyl)-3-ethylcarbodiimide hydrochloride 71 mg and dimethylaminopyridine (23mg) were added and allowed to react for 24 hours at room temperature. Following the reaction, the product was purified by silica gel chromatography (chloroform : methanol = 97 : 3) to yield 150 mg of Compound 3 (niraparib HAP). ([M + H] ⁺ : calculated value 488.21, observed value 488.3)

### (b) In vitro study 1

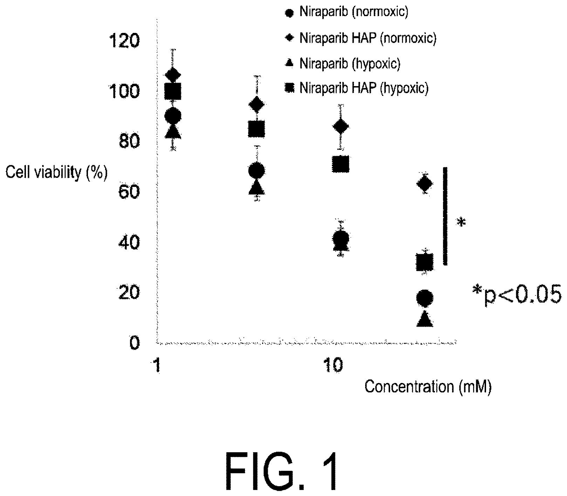

Human breast cancer cells (MDA-MB-231) were seeded in a 96-well plate at a density of 3000 cells/well, niraparib or Compound 3 was added, and the cell viability was observed after incubation at normal oxygen concentration and low oxygen concentration (0.2 %) for 2 days. The results are shown in FIG.1. From FIG. 1, it can be confirmed that the cytotoxicity is recovered at the low oxygen concentration.

### (c) In vitro study 2

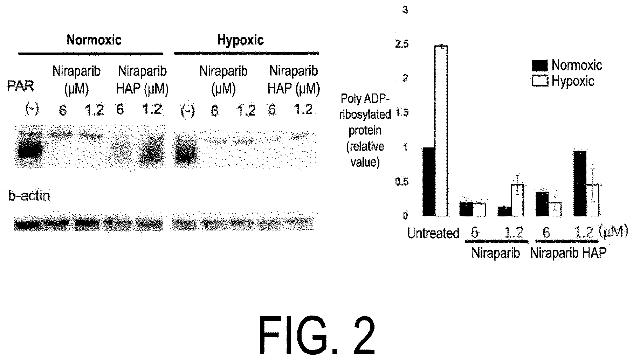

To determine whether the targeted PARP activity was inhibited under a hypoxic environment, proteins were recovered from the cells and poly ADP ribosylated (PARylated) proteins were detected by Western blotting. The results are shown in FIG. 2. From FIG. 2, it can be confirmed that PARP activity is specifically inhibited under the low oxygen environment.

### (d) In vivo study

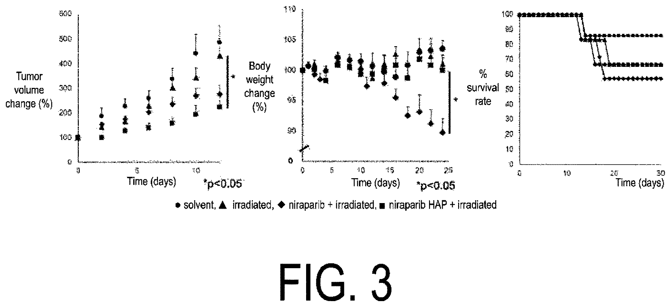

Balb/c nude mice (6 or 7 mice per group) were each subcutaneously implanted with MDA-MB-231 cells, and administered with niraparib or niraparib HAP to analyze the synergistic effect with radiotherapy. The results are shown in FIG. 3. From FIG. 3, it can be confirmed that the tumor growth inhibitory effect is enhanced in the drug-administered group as compared with the case of the radiation monotherapy. In addition, it can be confirmed that the niraparib-HAP-administered group showed no decrease in body weight and improved survival rate as compared with the niraparib-administered group.

### <Usage and dosage of a drug, and a dose of irradiation>

A drug: the drug was orally administered (Day 0, 1, 2, 7, 8, 9 (50 mg/kg), 10, 11, 14, 15, 16, 17, 18, 21, 22, 23, 24, 25 (100 mg/kg))
Irradiation: 1 hour of drug administration followed by irradiation at 0.5 Gy

### Example 2: Crizotinib HAP

### (a) Example of production

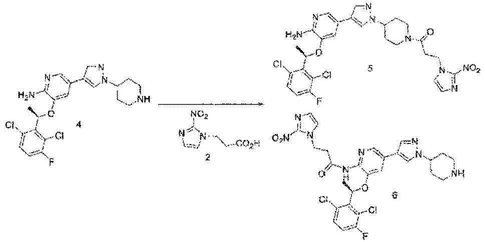

Crizotinib (Compound 4, 50 mg) was dissolved in dichloromethane 2 mL, Compound 2 (32 mg), 1-(3-dimethylaminopropyl)-3-ethylcarbodiimide hydrochloride 22 mg and dimethylaminopyridine (7 mg) were added and allowed to react for 24 hours at room temperature. Following the reaction, the product was purified by silica gel chromatography (chloroform : methanol = 97 : 3) to yield a mixture of compounds 5 and 6 (crizotinib HAP) by 20 mg. ([M + H] ⁺: calculated value 618.2, observed value 618.3)

### (b) In vitro study

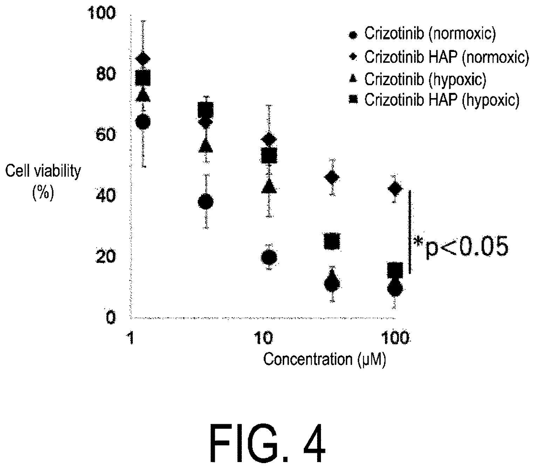

EML-ALK mutant human non-small-cell lung cancer (EMI,4-ALK fusion-A549) (ATCC Model No. CCL-185IG) was seeded in a 96-well plate at a density of 3000 cells/well, and the obtained crizotinib or crizotinib HAP (a mixture of 5 and 6) was added and incubated for 1 day at normal and low oxygen concentrations (0.2 %), followed by medium exchange and further cultured for 2 days under the same conditions to examine the cell viability. The results are shown in FIG. 4. From FIG. 4, it can be confirmed that cytotoxicity is recovered at the low oxygen concentration.

### Example 3: Dabrafenib HAP

### (a) Example of production

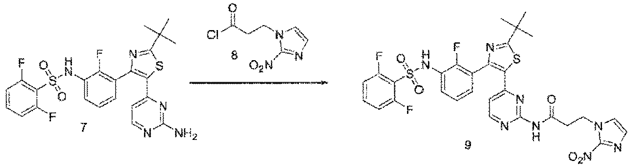

Dabrafenib (Compound 7, 50 mg) was dissolved in dichloromethane 2 mL, and Compound 8 (35 mg) was added, and allowed to react for 24 hours at room temperature . Following the reaction, the product was purified by silica gel chromatography (chloroform : methanol = 98 : 2) to yield Compound 9 (dabrafenib HAP) by 20mg. ([M - H]⁻: calculated value 685.1, observed value 685.3)

### (b) In vitro study

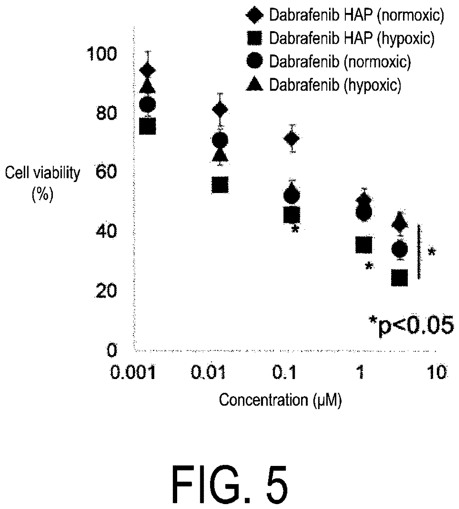

Human malignant melanomas (A375) were seeded in a 96-well plate at a density of 3000 cells/well, the obtained dabrafenib or Compound 9 was added, and the cell viability was observed when they were incubated for 2 days at normal and lower (0.2 %) oxygen concentrations. The results are indicated in FIG. 5. From FIG. 5, it can be confirmed that cytotoxicity is recovered at the low oxygen concentration.

### Example 4 : Vemurafenib HAP

### (a) Example of production

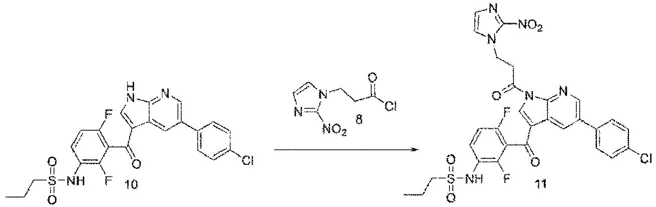

Vemurafenib (Compound 10, 50 mg) was dissolved in dichloromethane 2 mL and Compound 8 (35 mg) was added and allowed to react for 24 hours at room temperature. Following the reaction, purification was performed by silica gel chromatography (chloroform : methanol = 100 : 0 to 98 : 2) to yield Compound 11 (vemurafenib HAP) by 20 mg. ([M - H]⁻: calculated value 655.0, observed value 654.9)

### (b) In vitro study

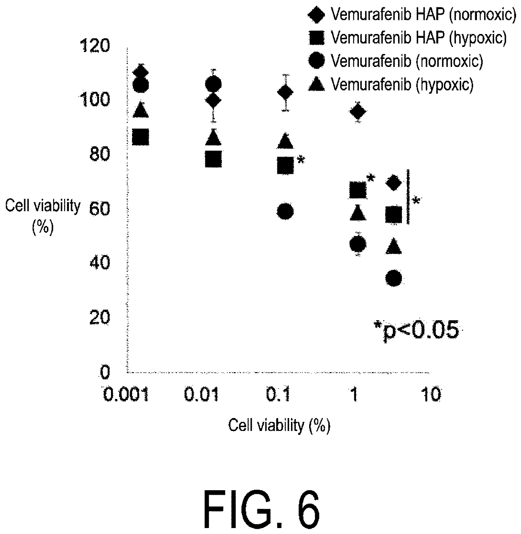

Human malignant melanoma (A375) cells were seeded in a 96-well plate at a density of 3000 cells/well, added with the obtained vemurafenib or Compound 11, and incubated for 2 days at normal and lower (0.2 %) oxygen concentrations to observe the cell viability. The results are shown in FIG. 6. From FIG. 6, it can be confirmed that cytotoxicity is recovered at the low oxygen concentration.

### Example 5: Entinostat HAP

### (a) Example of production

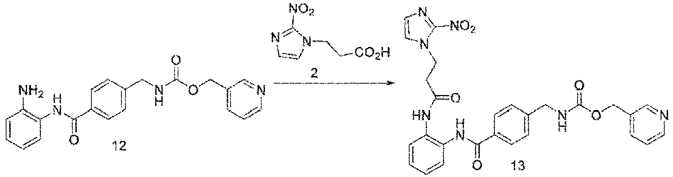

Entinostat (Compound 12, 100 mg) was dissolved in dichloromethane 2 mL, and Compound 2 (105 mg), 1-(3-dimethylaminopropyl)-3-ethylcarbodiimide hydrochloride 76 mg and dimethylaminopyridine (24 mg) were added and allowed to react for 24 hours at room temperature. Following the reaction, the product was purified by silica gel chromatography (chloroform : methanol = 97 : 3) to yield Compound 13 (entinostat HAP) by 50 mg. ([M + H]⁺: calculated value 544.2, observed value 544.5)

### (b) In vitro study

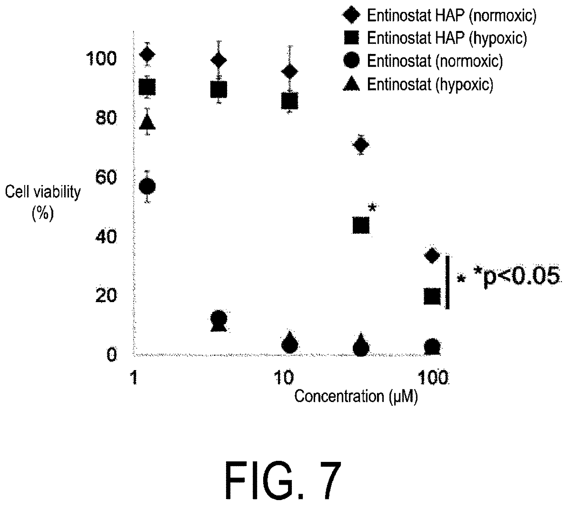

Human breast cancer cells (MDA-MB-231) were seeded in a 96-well plate at a density of 3000 cells/well, added with the obtained entinostat or Compound 13, and incubated for 4 days at normal and lower (0.2%) oxygen concentrations to observe the cell viability. The results are shown in FIG. 7. From FIG. 7, it can be confirmed that cytotoxicity is recovered at the low oxygen concentration.

### Example 6: Cobimetinib HAP

### (a) Example of production

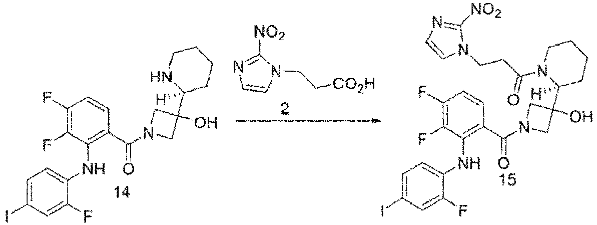

Cobimetinib (compound 14, 50 mg) was dissolved in dichloromethane 2 mL, Compound 2 (26 mg), 1-(3-dimethylaminopropyl)-3-ethylcarbodiimide hydrochloride 28 mg and dimethylaminopyridine (9 mg) were added, and allowed to react for 24 hours at room temperature. Following the reaction, the product was purified by silica gel chromatography (chloroform : methanol = 100 : 0 to 97 : 3) to yield Compound 15 (cobimetinib HAP) by 30 mg.

### ([M - H]⁻: calculated value 697.1, observed value 696.9)

### (b) In vitro study

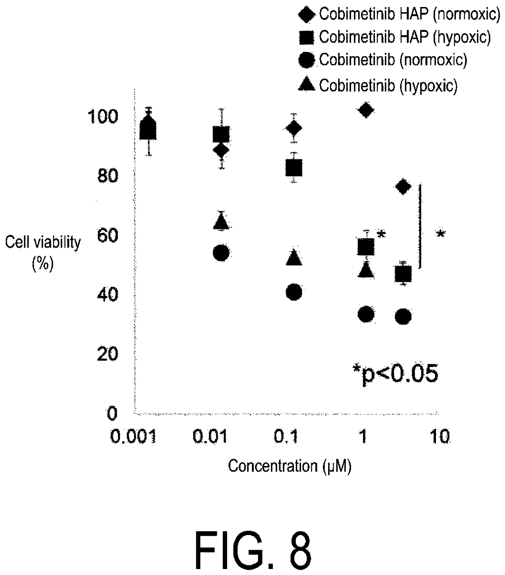

Human malignant melanomas (A375) were seeded in a 96-well plate at a density of 3000 cells/well, and the obtained cobimetinib or Compound 15 was added and incubated for 2 days at normal and lower (0.2 %) oxygen concentrations to observe the cell viability. The results are shown in FIG. 8. From FIG. 8, it can be confirmed that cytotoxicity is recovered at the low oxygen concentration.

### Example 7: Palbociclib HAP

### (a) Example of production

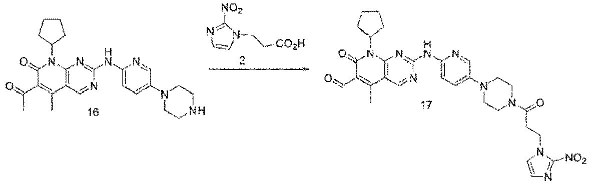

Palbociclib (Compound 16, 100 mg) was dissolved in dichloromethane 2 mL, and Compound 2 (75 mg), 1-(3-dimethylaminopropyl)-3-ethylcarbodiimide hydrochloride 51 mg and dimethylaminopyridine (16 mg) were added, and allowed to react for 24 hours at room temperature. Following the reaction, the product was purified by silica gel chromatography (chloroform : methanol = 100 : 0 to 97 : 3) to yield Compound 17 (palbociclib HAP) by 120 mg.

### ([M + H]⁺: calculated value 615.3, observed value 615.1)

### (b) In vitro study

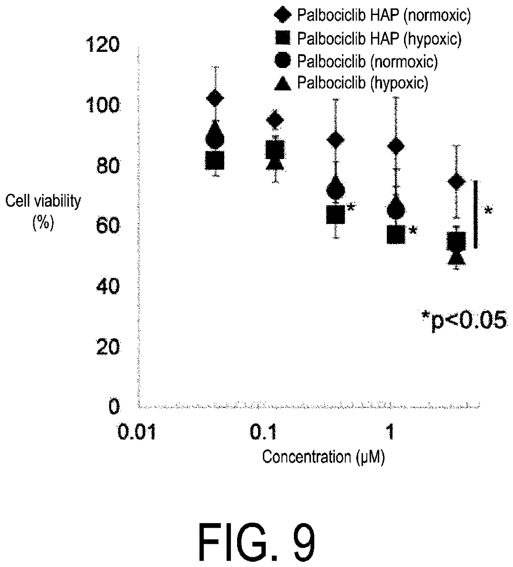

Human breast cancer cells (MCF-7) were seeded in a 96-well plate at a density of 3000 cells/well, added with the obtained palbociclib or Compound 17, and incubated for 2 days at normal and lower (0.2 %) oxygen concentrations to observe the cell viability. The results are shown in FIG. 9. From FIG. 9, it can be confirmed that cytotoxicity is recovered at the low oxygen concentration.

### (c) In vivo study

Human pancreatic cancer cells (MIA Paca-2) were subcutaneously implanted into Balb/c nude mice (6 mice per group), and palbociclib or palbociclib HAP was orally administered (100 mg/kg, twice/week) to examine the synergistic effect with radiotherapy (2 Gy on the day after drug administration, twice/week).

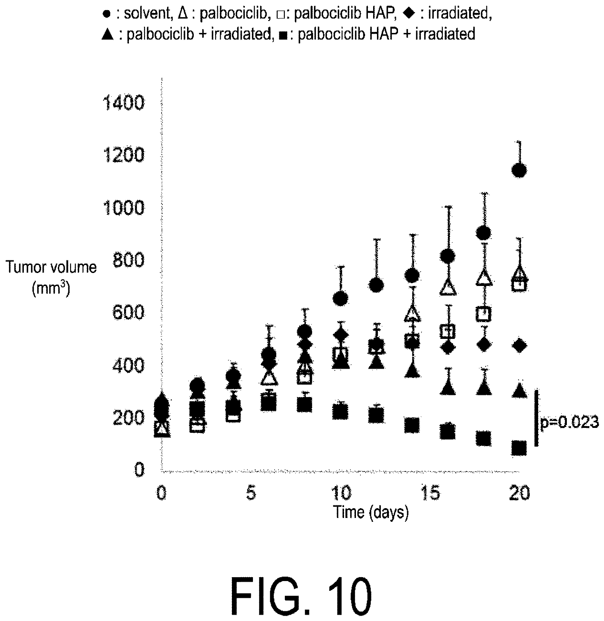

The results are shown in FIG. 10. From FIG. 10, it can be confirmed that in the prodrug-administered group, the tumor growth inhibitory effect was enhanced when radiation was used in combination, as compared with the case in which radiation therapy was used alone and the case in which the drug substance palbociclib and radiation were used in combination.

### Example 8: Ribociclib HAP

### (a) Example of production

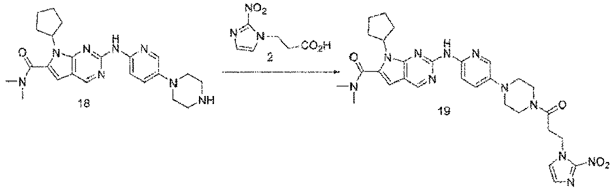

Ribociclib (Compound 18, 100 mg) was dissolved in dichloromethane 2 mL, Compound 2 (75 mg), 1-(3-dimethylaminopropyl)-3-ethylcarbodiimide hydrochloride 51 mg and dimethylaminopyridine (16 mg) were added, and allowed to react for 24 hours at room temperature. Following the reaction, the product was purified by silica gel chromatography (chloroform : methanol = 100 : 0 to 97 : 3) to yield Compound 19 (ribociclib HAP) by 100 mg.

### ([M + H]⁺: calculated value 602.3, observed value 602.2)

### (b) In vitro study

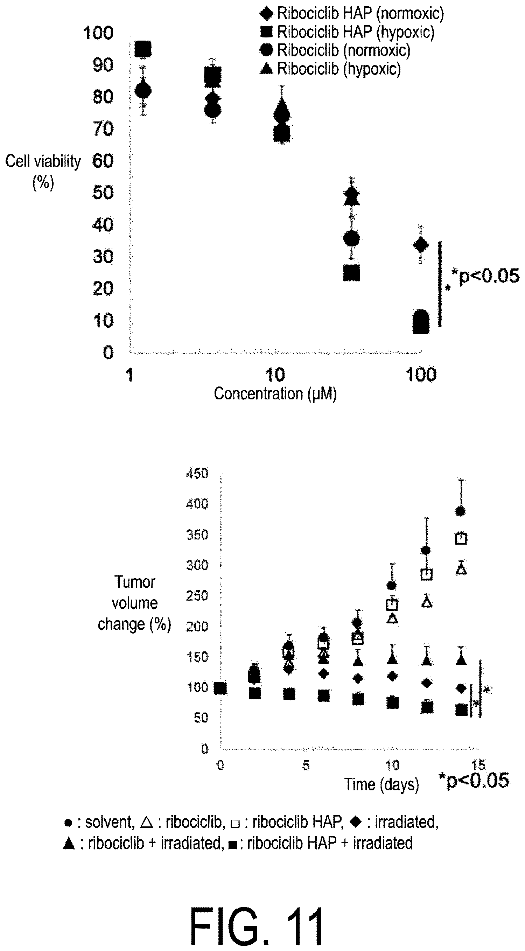

Human breast cancer cells (MCF-7) were seeded in a 96-well plate at a density of 3000 cells/well, the obtained ribociclib or Compound 19 was added, and the cells were incubated for 2 days at normal and low (0.2 %) oxygen concentrations to examine the cell viability. The results are illustrated in FIG. 11. From FIG. 11, it can be confirmed that cytotoxicity is recovered at the low oxygen concentration.

### (c) In vivo study

A 17β-estradiol sustained-release pellet (manufactured by Innovative Research of America) and human breast cancer cells (MCF7) were subcutaneously implanted to Balb/c nude mice (6 mice per group), and ribociclib or ribociclib HAP was orally administered (75 mg/kg, twice/week) to examine the synergistic effect with radiotherapy (1 Gy, on the day after drug administration, twice/week). The results are illustrated in a lower panel of FIG. 11. From this figure, it can be confirmed that in the prodrug-administered group, the tumor growth inhibitory effect was enhanced when radiation was used in combination, as compared with the case in which radiation therapy was used alone or the case in which the drug substance ribociclib was used in combination with radiation.

### Example 9: Gemcitabine HAP

### In vivo study 1:

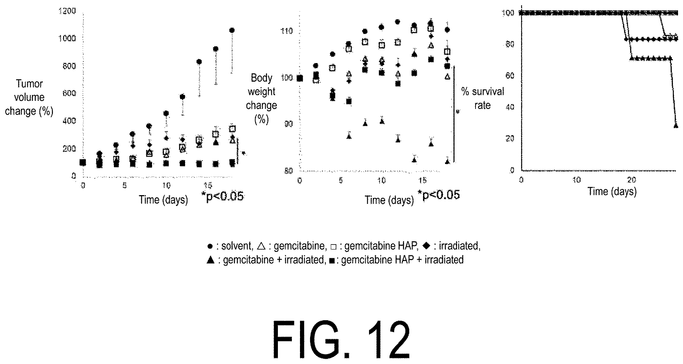

Human pancreatic cancer cells (MIA Paca-2) were subcutaneously implanted into Balb/c nude mice (6 or 7 mice per group), respectively, and gemcitabine or gemcitabine HAP (prepared according to the method described in Examples of JP 5676020 B2) was intraperitoneally administered (120 mg/kg, twice/week) to examine the synergistic effect of the combination therapy with radiotherapy (2 Gy, on the day after drug administration, twice/week). The results are shown in FIG. 12. From FIG. 12, it can be confirmed that the tumor growth inhibitory effect was enhanced in the drug-administered group as compared with the case of administrating the radiation monotherapy. In addition, as compared with the gemcitabine-administered group, the gemcitabine-HAP administered group showed no decrease in body weight and an improved survival rate.

### In vivo study 2:

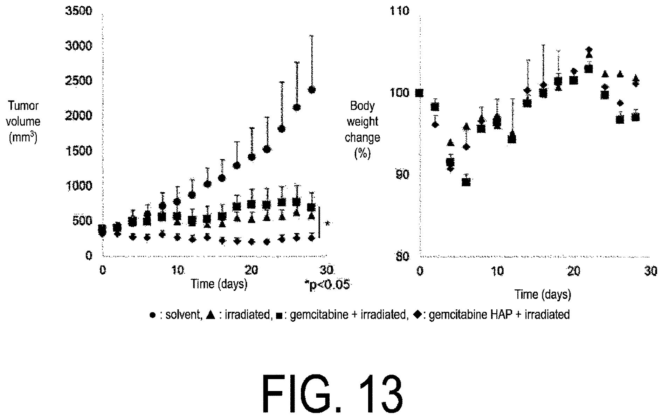

Human pancreatic cancer cells (MIA Paca-2) were subcutaneously implanted to Balb/c nude mice (6 mice per group), and gemcitabine or gemcitabine HAP was intraperitoneally administered (10 mg/kg, twice/week) to examine the synergistic effect of the combination therapy with radiotherapy (1 Gy, on the day after drug administration, twice/week). The results are shown in FIG. 13. From FIG. 13, it can be confirmed that the tumor growth inhibitory effect was enhanced in the gemcitabine-HAP administered group when radiation was used in combination, as compared with the case of radiation monotherapy and the case of using the drug substance gemcitabine and radiation in combination. When the dosage was reduced to 10 mg/kg, it can be confirmed that the effect was higher than that of gemcitabine when used in combination with radiation.

### In vivo study 2 (part 2):

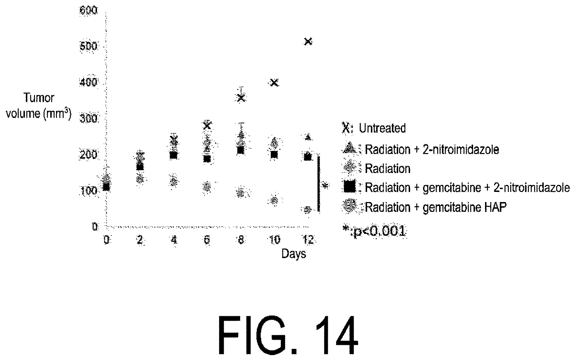

Human cancer cells (MIA Paca-2) were subcutaneously implanted into Balb/c nude mice (7 mice per group), respectively, and 2-nitroimidazole (10 mg/kg, twice/week), 2-nitroimidazole and gemcitabine (10 mg/kg respectively, twice/week), and gemcitabine HAP (10 mg/kg, twice/week) were intraperitoneally administered to examine the synergistic effect of the combination therapy with irradiation (1 Gy, on the day after drug administration, twice/week). The results are shown in FIG. 14. From FIG. 14, it can be confirmed that the tumor growth inhibitory effect was enhanced in the gemcitabine-HAP administered group when radiation was used in combination, as compared with the case of radiation monotherapy and the case of using 2-nitroimidazole which is a radiation sensitizer and the case of using gemcitabine which is a drug substance, 2-nitroimidazole and radiation in combination.

### In vivo study 3:

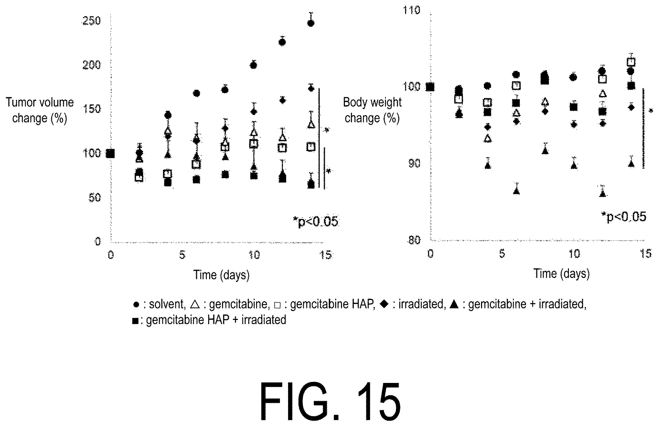

Human cholangiocarcinoma cells (HuCCT1) were subcutaneously implanted into Balb/c nude mice (5 mice per group), respectively, and gemcitabine or gemcitabine HAP was intraperitoneally administered (30 mg/kg, twice/week), respectively to examine the synergistic effect of the combination therapy with radiotherapy (2 Gy, on the day after drug administration, twice/week). The results are shown in FIG. 15. From FIG. 15, it can be confirmed that the tumor growth inhibitory effect was enhanced in the drug-administered group as compared with that of radiation monotherapy. In addition, as compared with the gemcitabine-administered group, the gemcitabine-HAP administered group did not show a decrease in body weight.

### Example 10: Doxorubicin HAP

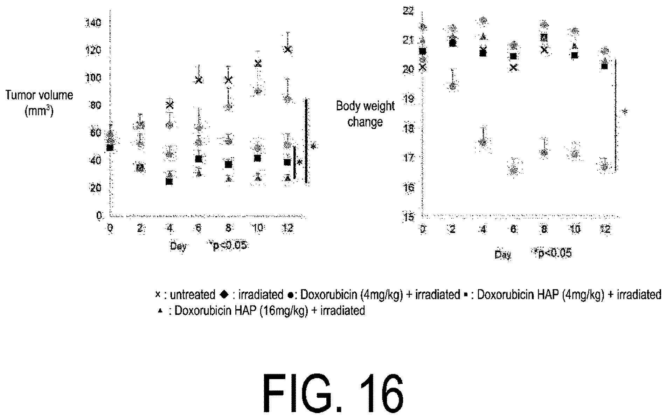

Balb/c nude mice (6 mice or more per group) were subcutaneously implanted with 17β-estradiol sustained-release pellets (produced by Innovative Research of America) and human breast cancer cells (MCF7), and intraperitoneally administered with doxorubicin (4 mg/kg, Day 0 and 2) or doxorubicin HAP 4 mg/kg, or 16 mg/kg, Day 0, 2, 7 and 9) to examine the synergistic effect with radiotherapy (0.5 Gy, Day 1, 3, 8 and 10). The results are shown in a lower panel of FIG. 16. From this figure, it can be confirmed that in the prodrug-administered group, the tumor growth inhibitory effect was enhanced when radiation was used in combination, as compared with the case in which radiotherapy was used alone and the case in which the drug substance doxorubicin was used in combination with radiation. In addition, as compared with the doxorubicin-administered group, the doxorubicin HAP-administered group did not show the weight loss.

## Claims

1. A pharmaceutical formulation for improving or enhancing, or for use in improving or enhancing, a therapeutic effect of combination therapy including radiotherapy, in the combination therapy of radiotherapy and drug therapy for a malignant tumor, the pharmaceutical formulation comprising a compound represented by Formula I as an active ingredient: 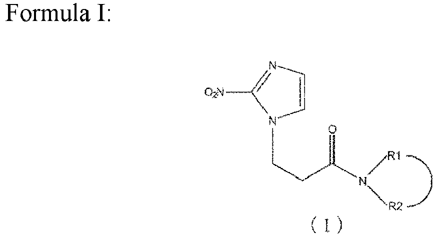 wherein,
a moiety in which R1 and R2 bond to an N atom is a part of an anticancer drug having an amino group or an imino group with the amino group or the imino group removed; in case of the anticancer drug having the amino group, either R1 or R2 represents a hydrogen atom, and the anticancer drug having the moiety in which R1 and R2 bond to an N atom is selected from the group consisting of: anthracyclines selected from doxorubicin, idarubicin, epirubicin, daunorubicin, pirarubicin, amrubicin, aclacinomycin, anthramycin and zorubicin; peptides selected from bleomycin and actinomycin; quinoline alkaloids selected from camptothecin, topotecan and irinotecan; taxanes selected from docetaxel and paclitaxel; vinca alkaloids selected from vinorelbine, vincristine, vinblastine and vindesine; deoxycytidines selected from gemcitabine and cytarabine; pyrimidines selected from 5-fluorouracil, capecitabine and doxifluridine; purine ring derivatives selected from fludarabine, 6-mercaptopurine and 6-thioguanine; macrolides selected from epothilone; and other anticancer drugs selected from niraparib, crizotinib, dabrafenib, vemurafenib, entinostat, panobinostat, cobimetinib, palbociclib and ribociclib.

2. The pharmaceutical formulation according to claim 1, wherein the compound represented by Formula I is derived from the following anticancer drug and represented by the following structural formula,
the compound being: derived from gemcitabine and represented by the structural formula: 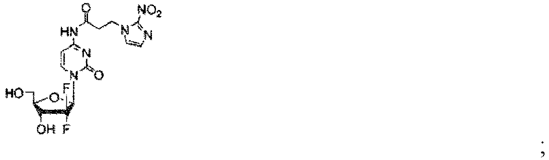
derived from doxorubicin and represented by the structural formula: 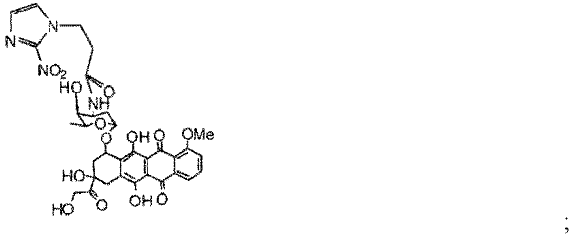
derived from 5-fluorouracil and represented by any one of the structural formulae: 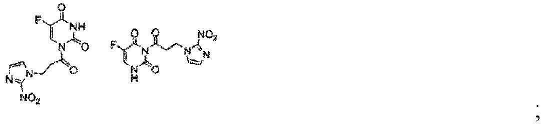
derived from niraparib and represented by the structural formula: 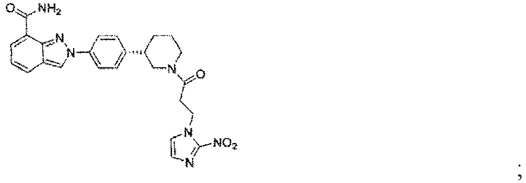
derived from crizotinib and represented by any one of the structural formulae: 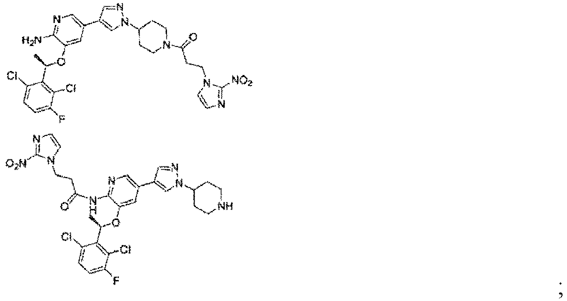
derived from dabrafenib and represented by the structural formula: 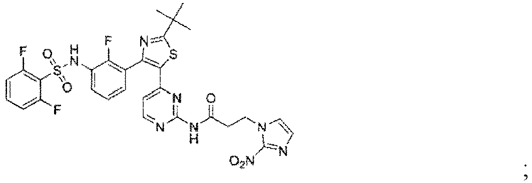
derived from vemurafenib and represented by the structural formula: 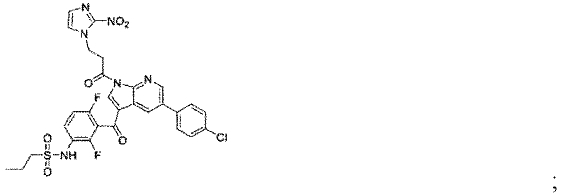
derived from entinostat and represented by the structural formula: 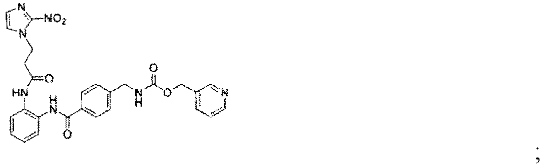
derived from cobimetinib and represented by the structural formula: 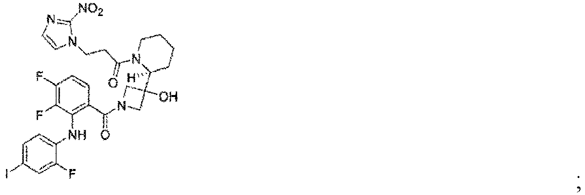
derived from palbociclib and represented by the structural formula: 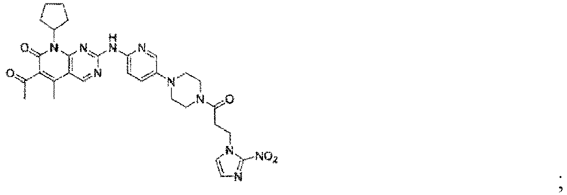 or
derived from ribociclib and represented by the structural formula:

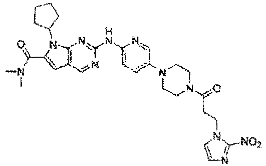

3. Combination therapy of drug therapy and radiotherapy for a malignant tumor, the combination therapy comprising: administering an effective dose of a compound represented by Formula I to a patient or an individual in need of treatment for a malignant tumor; and irradiating the patient or the individual with an effective dose of radiation: 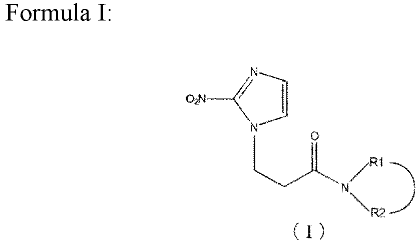 wherein,
a moiety in which R1 and R2 bond to an N atom is a part of an anticancer drug having an amino group or an imino group with the amino group or the imino group removed; and in case of the anticancer drug having the amino group, either R1 or R2 represents a hydrogen atom, and the anticancer drug having the moiety in which R1 and R2 bond to an N atom is selected from the group consisting of: anthracyclines selected from doxorubicin, idarubicin, epirubicin, daunorubicin, pirarubicin, amrubicin, aclacinomycin, anthramycin and zorubicin; peptides selected from bleomycin and actinomycin; quinoline alkaloids selected from camptothecin, topotecan and irinotecan; taxanes selected from docetaxel and paclitaxel; vinca alkaloids selected from vinorelbine, vincristine, vinblastine and vindesine; deoxycytidines selected from gemcitabine and cytarabine; pyrimidines selected from 5-fluorouracil, capecitabine and doxifluridine; purine ring derivatives selected from fludarabine, 6-mercaptopurine and 6-thioguanine; macrolides selected from epothilone; and other anti-cancer drugs selected from niraparib, crizotinib, dabrafenib, vemurafenib, entinostat, panobinostat, cobimetinib, palbociclib and ribociclib.

4. The combination therapy according to claim 3, wherein the compound represented by Formula I is derived from the following anticancer drugs and is represented by the following structural formulae:
the compound being: derived from gemcitabine and represented by the structural formula: 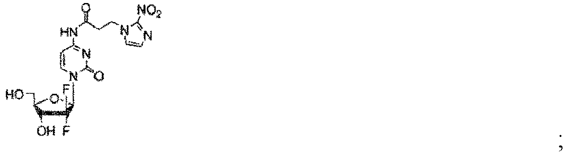
derived from doxorubicin and represented by the structural formula: 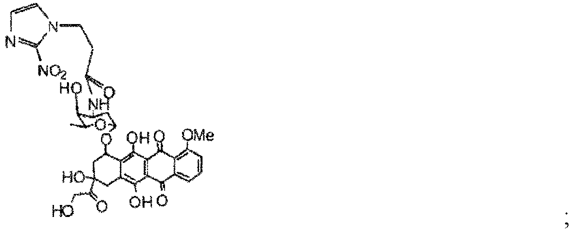
derived from 5-fluorouracil and represented by any one of the structural formulae: 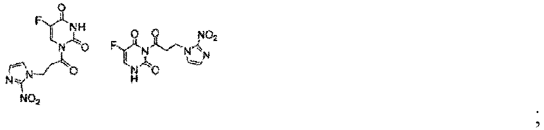
derived from niraparib and represented by the structural formula: 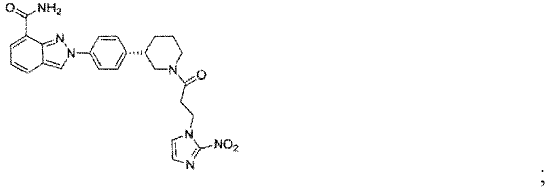
derived from crizotinib and represented by any one of the structural formulae: 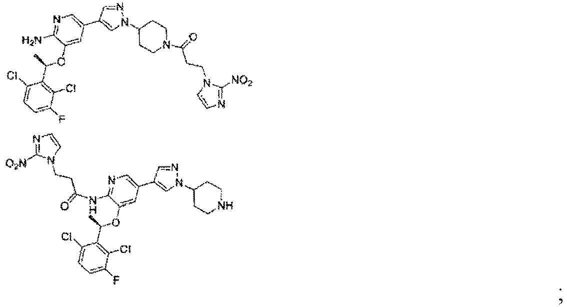
derived from dabrafenib and represented by the structural formula: 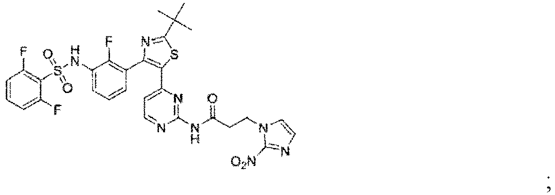
derived from vemurafenib and represented by the structural formula: 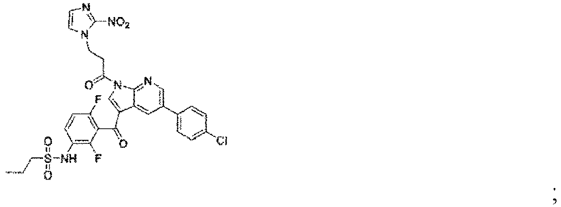
derived from entinostat and represented by the structural formula: 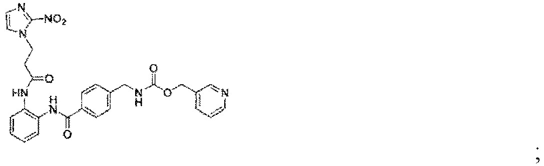
derived from cobimetinib and represented by the structural formula: 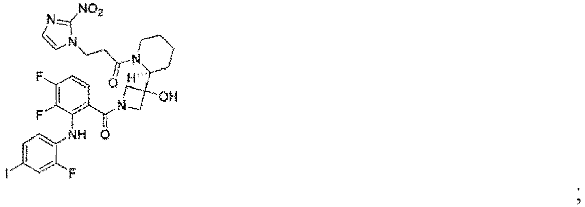
derived from palbociclib and represented by the structural formula: 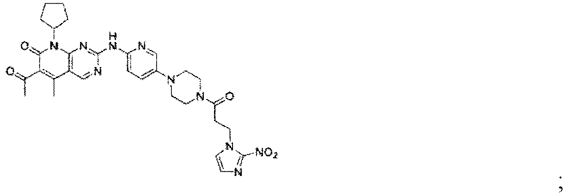 or
derived from ribociclib and represented by the structural formula:

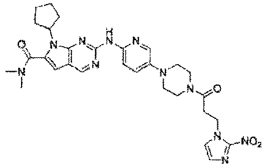

5. A compound represented by Formula I-1 or a pharmaceutically acceptable salt thereof: 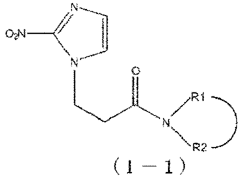 wherein,
a moiety in which R1 and R2 bond to an N atom is a part of an anticancer drug having an amino group or an imino group with the amino group or the imino group removed; and in case of the anticancer drug having the amino group, either R1 or R2 represents a hydrogen atom, and the compound is derived from the anticancer drug selected from the group consisting of niraparib, crizotinib, dabrafenib, vemurafenib, entinostat, cobimetinib, palbociclib and ribociclib; respectively, the compound being: derived from niraparib and represented by the following structural formula: 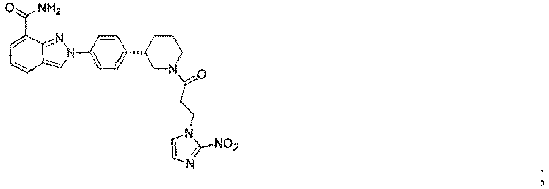
derived from crizotinib and represented by any one of the structural formulae: 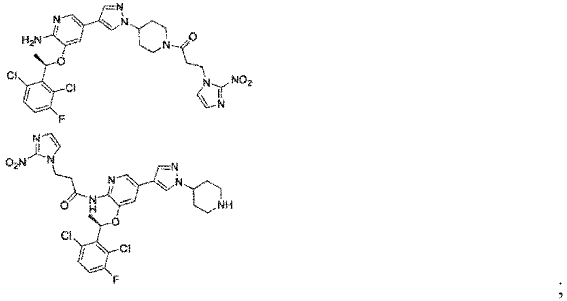
derived from dabrafenib and represented by the structural formula: 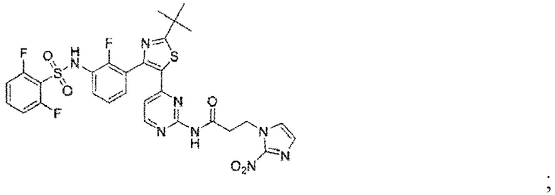
derived from vemurafenib and represented by the structural formula: 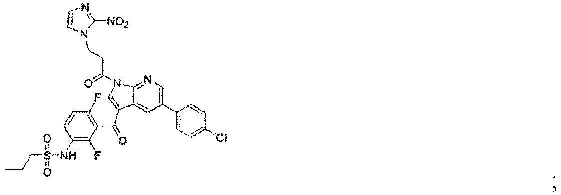
derived from entinostat and represented by the structural formula: 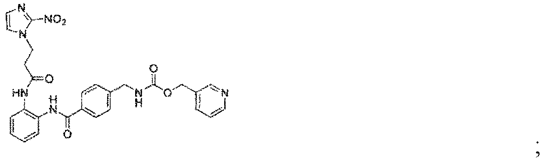
derived from cobimetinib and represented by the structural formula: 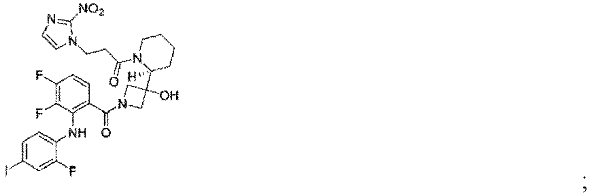
derived from palbociclib and represented by the structural formula: 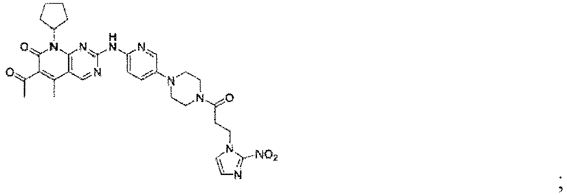 or
derived from ribociclib and represented by the structural formula:

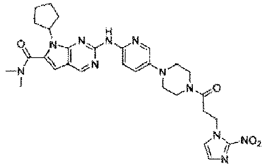

6. A method of providing a hypoxia-responsive prodrug in combination therapy of radiotherapy and drug therapy for a malignant tumor, for improving or enhancing a therapeutic effect of combination therapy, the method comprising:
(1) covalently bonding an anticancer drug, via an amino group (NH₂) or an imino group (-NH-) with an imidazolepropionic acid represented by Formula II: 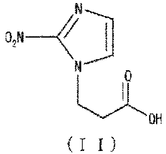 to form a prodrug of the anticancer drug, and
(2) treating a tumor experimental animal with the prodrug of the anticancer drug obtained in step (1) and its corresponding parent compound in combination with radiotherapy, to select the prodrug having statistically significantly lower side effects or significantly higher therapeutic effects than radiotherapy alone.
